# EUROPÄISCHE PATENTANMELDUNG

(11) **EP 3 346 000 A1**
(43) Veröffentlichungstag der Anmeldung: **11.07.2018**
(21) Anmeldenummer: 17150489.7
(22) Anmeldetag: 05.01.2017
(51) Int. Cl.: C12N 5/077

(54) **VERFAHREN ZUR SELEKTION VON BANDSCHEIBENZELLEN**

(71) Anmelder: co.don AG, 14513 Teltow (DE); Charité - Universitätsmedizin Berlin, 10117 Berlin (DE)
(72) Erfinder: SITTINGER, Michael, 12305 Berlin (DE); RINGE, Jochen, 16547 Birkenwerder (DE); SMINK, Jeske Johanna, 10437 Berlin (DE); SCHUBERT, Ann-Kathrin, 12103 Berlin (DE)
(74) Vertreter: Simandi, Claus

(57) **Zusammenfassung**

Die Erfindung betrifft ein Verfahren zur Selektion von Bandscheibenzellen mittels Markern, insbesondere zur Unterscheidung von Bandscheibenzellen und zwar Zellen aus Nucleus Pulposus und Zellen aus Annulus Fibrosus. Weiterhin betrifft die Erfindung die Vermehrung oder Anreicherung von selektierten Zellen und deren Zelltransplantation, insbesondere zur Regeneration der Bandscheibe.

## Beschreibung

Die Erfindung betrifft ein Verfahren zur Selektion von Bandscheibenzellen mittels Markern, insbesondere zur Unterscheidung von Bandscheibenzellen und zwar Zellen aus Nucleus Pulposus (kurz: NP) und Zellen aus Annulus Fibrosus (kurz: AF).

Weiterhin betrifft die Erfindung die Vermehrung oder Anreicherung von selektierten Zellen und deren Zelltransplantation, insbesondere zur Regeneration der Bandscheibe.

Die Bereitstellung von geeigneten Bandscheibenzellen für den Menschen ist eine medizinische Herausforderung, sei es zu Zwecken im therapeutischen in-vivo Einsatz im Wege der Zelltransplantation oder in der Entwicklung von in-vitro Zellsystemen einschließlich zugehöriger Zellkulturen zur gezielten Anreicherung selektionierter Bandscheibenzellen.

Die Bandscheibe (auch: Discus intervertebralis) ist aus zwei Gewebetypen aufgebaut und zwischen zwei Wirbelkörpern zu lokalisieren (sogenannter Zwischenwirbelraum). Histologischanatomisch ist die Bandscheibe eine Wirbelsychondrose und von knorpelartiger Natur. Im Inneren befindet sich ein erstes Gewebe aus einem gallertartigen Kern (Nucleus Pulposus), der von einem zweiten Gewebe, einem faserartigen Ring (Annulus Fibrosus), umschlossen und geschützt wird.

Bei einer degenerativen Erkrankung eines Bewegungssegments der Wirbelsäule, wie z.B. ein Bandscheibenvorfall (hernia) ist der äußere AF beschädigt und der innere NP tritt aus. Das hernierte Gewebe drückt auf den angrenzenden Nerv und versursacht Schmerzen. Bei andauernden Symptomen wird dem Patienten das hernierte Gewebe (NP) operativ entfernt um den gequetschten Nerv zu entlasten (sogenannte Diskektomie) und insbesondere eine Spinalkanalstenose zu verhindern.

Da die Bandscheibe, wie alle knorpelartigen Gewebe, nicht in der Lage ist sich eigenständig zu regenerieren, bleibt die Bandscheibe ohne weitere Therapiemaßnahmen irreversibel geschädigt und in ihrer Funktion beeinträchtigt.

An dieser Stelle gilt der Einsatz von zellbasierten Therapien bei der Behandlung von Bandscheibenschäden als vielversprechende Methode um eine geschädigte Bandscheibe zu regenerieren und deren Funktion wieder herzustellen.

Die Anmelderin verwendet bereits zellbasierte Therapieverfahren (chondrotransplant® DISC) zur Regeneration der Bandscheibe, siehe WO2005055877A2 der Anmelderin.

Als Ausgangsgewebe kann das eigene Gewebe des Patienten/Spenders aus dem Bandscheibenvorfall dienen oder aus anderen Körperregionen, die Knorpel enthalten (z.B. Nase, Knie).

Aus dem Gewebe werden ex vivo Zellen isoliert und in vitro expandiert bevor sie dem Patienten in Suspension oder zusammen mit einem Biomaterial in die Bandscheibe gespritzt werden (sogenannte Bandscheiben - Zelltransplantation (ADCT, autologous disc-derived chondrocyte transplantation)). Dieses Verfahren, die körpereigene Bandscheiben-Zelltransplantation, kann die typischerweise nach einem Bandscheibenvorfall auftretenden, fortschreitenden Abbauprozesse der betroffenen Bandscheibe aufhalten und somit einer weiteren Degeneration der Bandscheibe als auch der Wirbelsäule entgegenwirken. Das Verfahren kann minimal-invasiv und routinemäßig erfolgen.

Allerdings besteht ein hohes Bedürfnis an einer Qualitätssicherung als auch an einer Patientensicherheit. Beispielsweise sollte das entnommene Gewebe keine Stammzellen enthalten, die im Fall der Transplantation oder Re-Implantierung eine Tumorigenität aufweisen können. Ebenfalls ist jedwedes Fremdgewebe zu vermeiden, wie Verunreinigungen aus Knochenzellen z.B. aus einer einhergehenden Osteochondrose.

Im Stand der Technik sind Bandscheibenzellen im Tiermodell beschrieben (Lee, C.R., et al., A phenotypic comparison of intervertebral disc and articular cartilage cells in the rat. Eur Spine J, 2007. 16(12): p. 2174-85; Clouet, J., et al., Identification of phenotypic discriminating markers for intervertebral disc cells and articular chondrocytes. Rheumatology (Oxford), 2009. 48(11): p. 1447-50; Tang, X., L. Jing, and J. Chen, Changes in the molecular phenotype of nucleus pulposus cells with intervertebral disc aging. PLoS One, 2012. 7(12): p. e5202).

Die bekannten Knorpelmarker, wie Collagen Typ 2, Aggrecan oder SOX9 dienen vorrangig zur Darstellung des hyalinen Charakters von Gelenkknorpel (articular cartilage, AC) und sind nicht ausreichend geeignet für eine Aussage hinsichtlich der Eignung zur Zelltransplantation samt erforderlicher Zellidentität. Bei den ebenfalls frühzeitig festgelegten Bandscheibenspezifischen Markern Versican, Biglycan, Decorin und Fibromodulin handelt es sich um sekretierte Matrixproteine, die mehr den biochemischen und makromolekularen Aufbau der Bandscheibe beschreiben als eine Aussage zur Eignung zur Zelltransplantation samt Zellidentität erlauben (Choi, H., Z.I. Johnson, and M.V. Risbud, Understanding nucleus pulposus cell phenotype: a prerequisite for stem cell based therapies to treat intervertebral disc degeneration. Curr Stem Cell Res Ther, 2015. 10(4): p. 307-16).

Es beschreiben bisher nur wenige Autoren die Diversität der Zelltypen in der humanen Bandscheibe auf molekularer Ebene (Mwale, F., P. Roughley, and J. Antoniou, Distinction between the extracellular matrix of the nucleus pulposus and hyaline cartilage: a requisite for tissue engineering of intervertebral disc. Eur Cell Mater, 2004. 8: p. 58-63; discussion 63-4, Pattappa, G., et al., Diversity of intervertebral disc cells: phenotype and function. J Anat, 2012. 221(6): p. 480-96) und zeigen hoch-exprimierte Gene in humanen NP-Gewebe (Rutges, J., et al., Variations in gene and protein expression in human nucleus pulposus in comparison with annulus fibrosus and cartilage cells: potential associations with aging and degeneration. Osteoarthritis Cartilage, 2010. 18(3): p. 416-23) oder AF-Gewebe (Gruber, H.E., et al., Microarray analysis of laser capture microdissected-anulus cells from the human intervertebral disc. Spine (Phila Pa 1976), 2007. 32(11): p. 1181-7; van den Akker, G.G., et al., Novel immortal human cell lines reveal subpopulations in the nucleus pulposus. Arthritis Res Ther, 2014. 16(3): p. R135), jedoch liegt der Fokus vorrangig auf den Merkmalen der Degeneration der Bandscheibe und nicht auf der Eignung zur Regeneration der Bandscheibenzellen.

Des Weiteren berichten Risbud MV et al. (Risbud, M.V., et al., Defining the phenotype of young healthy nucleus pulposus cells: recommendations of the Spine Research Interest Group at the 2014 annual ORS meeting. J Orthop Res, 2015. 33(3): p. 283-93) über "phänotypische Marker", die jedoch überwiegend auf Studien mit tierischem Bandscheibengewebe beruhen. Im Tiermodell beschriebene Ergebnisse lassen sich jedoch nicht vollständig auf das humane System übertragen (Minogue, B.M., et al., Transcriptional profiling of bovine intervertebral disc cells: implications for identification of normal and degenerate human intervertebral disc cell phenotypes. Arthritis Res Ther, 2010. 12(1): p. R22). Zudem wird das Genexpressionsprofil von Bandscheibenzellen nachweislich durch Kulturbedingungen wie Sauerstoffgehalt, Medienzusammensetzung oder Passagenzahl beeinflusst (Kluba, T., et al., Human anulus fibrosis and nucleus pulposus cells of the intervertebral disc: effect of degeneration and culture system on cell phenotype. Spine (Phila Pa 1976), 2005. 30(24): p. 2743-8).

Es besteht daher die Aufgabe ein Verfahren zur Selektion von humanen und nativen Bandscheibenzellen bereitzustellen, so dass insbesondere keine Fremdzellen in-vitro angereichert und reimplantiert werden und eine effektive Regeneration der defekten Bandscheibe erfolgt.

Weiterhin besteht die Aufgabe die geeignete Zellidentität oder Zellspezifität der entnommenen Bandscheibenzellen zu gewährleisten.

Eine weitere Aufgabe besteht darin die Bandscheibenzellen aus NP- und AF-Gewebe zu unterscheiden. Dies erlaubt die spezifische Anreicherung oder Vermehrung von NP- oder AF-Zellen und deren Re-Implantierung bzw. Transplantation.

Überraschender Weise haben die Erfinder gefunden, dass bestimmte Marker geeignet sind, Bandscheibenzellen zu identifizieren bzw. zu charakterisieren und entsprechend zur Selektion von Bandscheibenzellen einzusetzen, so dass eine effiziente Regeneration der Bandscheibe ermöglicht wird.

Die Aufgabe wird daher durch ein Verfahren zur Selektion von humanen und nativen Bandscheibenzellen gelöst, wobei mindestens ein Marker ausgewählt aus der Gruppe

| CODE | Name | Gene ID | NCBI Reference Sequence: |
|---|---|---|---|
| ARAP2 | ArfGAP with RhoGAP domain, ankyrin repeat and PH domain 2 | 116984 | NM_015230 |
| CDKN2B | cyclin-dependent kinase inhibitor 2B (p15, inhibits CDK4) | 1030 | NM_004936/ NM_078487 |
| DEFB1 | defensin, beta 1 | 1672 | NM_005218 |
| DSC3 | desmocollin 3 | 1825 | NM_001941 / NM_024423 |
| ERFE | erythroferrone, frühere Bezeichnung FAM132B | 151176 | NM_001291832 |
| SPTLC3 | serine palmitoyltransferase, long chain base subunit 3 | 55304 | NM_018327 |
| ADGRL4 | adhesion G protein-coupled receptor L4 (EGF, latrophilin and seven transmembrane domain containing 1), frühere Bezeichnung ELTD1 | 64123 | NM_022159 |
| ANKRD29 | ankyrin repeat domain 29 | 147463 | NM_173505/ NM_001308238 |
| EMCN | endomucin | 51705 | NM_016242/ NM_001159694 |
| LDB2 | LIM domain binding 2 | 9079 | NM_001290/ NM_001130834/ NM_001304434/ NM_001304435 |
| OLFML2A | olfactomedin-like 2A | 169611 | NM_182487/ NM_001282715 |

bestimmt wird. Die vorstehenden erfindungsgemäßen Marker sind anhand der genannten Gene ID und NCBI Referenz zum Anmeldetag eindeutig zu identifizieren. Isoformen sind ebenfalls berücksichtigt und angegeben. Der Fachmann ist in der Lage aus diesen Angaben eine entsprechende cDNA als auch zugehöriges Peptid herzustellen.

Erfindungsgemäß werden die Bandscheibenzellen vorzugsweise aus dem Gewebe einer defekten Bandscheibe entnommen.

Die Bestimmung der Marker kann beispielsweise mittels einem Blot erfolgen, insbesondere mittels einem Northern-Blot. Bevorzugt ist jedoch der Nachweis der Genexpression der erfindungsgemäßen Marker mittels quantitativer Real-Time-PCR (qPCR), siehe Beispiele.

Die Bestimmung der Marker erfolgt mit dem entnommenen Gewebe der defekten oder gesunden Bandscheibe und Teile davon. In einem ersten Schritt kann das AF-Gewebe von dem NF-Gewebe getrennt werden. Sofern erforderlich kann das Gewebe portioniert werden. Erfindungsgemäß bevorzugt sind solche Marker, die eine hohe Expressionsrate in den Bandscheibenzellen aufweisen.

Solche Bandscheibenzellen werden selektioniert oder ausgewählt und angereichert oder vermehrt. Weiterhin sind solche Bandscheibenzellen aufgrund der durch die Marker festgelegte Zellspezifität und Zellidentität hochgradig zur Zelltransplantation geeignet, da eine Regeneration der Bandscheibe erfolgt. Folglich dient die Auswahl der Marker der Qualitätssicherung der humanen und nativen Bandscheibenzellen zu deren Selektion und Vermehrung als auch Anreicherung, insbesondere zur Herstellung von Transplantaten oder Implantaten.

Daher betrifft die Erfindung ebenfalls die Isolation von selektionierten Bandscheibenzellen und Herstellung von Fraktionen oder Implantaten oder Transplantaten bestehend aus angereicherten Bandscheibenzellen. Die Fraktionen, Implantate oder Transplantate weisen zumindest jeweils mehr als 70 % vorzugsweise mehr als 80 % an selektionierten Bandscheibenzellen auf.

Die Bestimmung der Marker kann ebenfalls während der Anreicherung und Vermehrung der Bandscheibenzellen in einem Kulturmedium erfolgen.

Dies erlaubt eine Anreicherung von Bandscheibenzellen, auch mittels mehrfacher Passagen, die eine hohe Expressionsrate der Marker aufweisen. Je höher die Expressionsrate der Marker in den Bandscheibenzellen ist, umso mehr eignet sich die Bandscheibenzellen zur Transplantation als auch zur Regeneration der defekten Bandscheibe.

In einer besonderen Ausführungsformen der Erfindung betrifft die Erfindung ein Verfahren zur Differenzierung zwischen NP-Bandscheibenzellen und AF-Bandscheibenzellen.
Die Differenzierung kann erfolgen indem auf NP-Bandscheibenzellen selektioniert wird, wobei mindestens ein Marker aus der Gruppe

| | |
|---|---|
| CDKN2B | cyclin-dependent kinase inhibitor 2B (p15, inhibits CDK4) |
| DEFB1 | defensin, beta 1 |
| DSC3 | desmocollin 3 |
| ERFE | erythroferrone, frühere Bezeichnung FAM132B |
| SPTLC3 | serine palmitoyltransferase, long chain base subunit 3 |

bestimmt wird.

Daher betrifft die Erfindung ein Verfahren zur Differenzierung zwischen NP-Bandscheibenzellen und AF-Bandscheibenzellen, wobei eine Bestimmung mindestens eines Markers ausgewählt aus der Gruppe CDKN2B, DEFB1, DSC3, ERFE, SPTLC3 erfolgt.

In einer bevorzugten Ausführungsform werden mindestens zwei Marker oder das gesamte Markerpanel bestimmt.

Eine Differenzierung zwischen NP-Zellen und AF-Zellen ist möglich, da die Genexpression in NP-Bandscheibenzellen höher ist als in AF-Bandscheibenzellen.

Dies erlaubt die spezifische Auswahl bzw. Selektion von NP-Zellen und deren Anreicherung und Vermehrung.

Weiterhin können erfindungsgemäß AF-Zellen selektioniert werden, wobei mindestens ein Marker aus der Gruppe

| | |
|---|---|
| ADGRL4 | adhesion G protein-coupled receptor L4 (EGF, latrophilin and seven transmembrane domain containing 1), frühere Bezeichnung ELTD1 |
| ANKRD29 | ankyrin repeat domain 29 |
| EMCN | endomucin |
| LDB2 | LIM domain binding 2 |
| OLFML2A | olfactomedin-like 2A |

bestimmt wird. Daher betrifft die Erfindung ein Verfahren zur Differenzierung zwischen NP-Bandscheibenzellen und AF-Bandscheibenzellen, wobei eine Bestimmung mindestens eines Markers ausgewählt aus der Gruppe ADGRL4, ANKRD29, EMCN, LDB2, OLFML2A erfolgt.

In einer weiteren bevorzugten Ausführungsform betrifft die Erfindung ein Verfahren zur Differenzierung zwischen NP-Bandscheibenzellen und AF-Bandscheibenzellen, wobei eine Bestimmung mindestens eines Markers ausgewählt aus der Gruppe CDKN2B, DEFB1, DSC3, ERFE, SPTLC3 erfolgt und eine Bestimmung mindestens eines Markers ausgewählt aus der Gruppe ADGRL4, ANKRD29, EMCN, LDB2, OLFML2A erfolgt.

Diese Ausführungsform erlaubt die sichere Unterscheidung zwischen NP-Bandscheibenzellen und AF-Bandscheibenzellen.

In einer bevorzugten Ausführungsform werden mindestens zwei Marker oder das gesamte Markerpanel bestimmt.
Weiterhin sind diese Marker für AF-Bandscheibenzellen spezifisch. Ergo, für NP-Zellen negativ. Folglich können vorteilhaft AF-Bandscheibenzellen von NP-Bandscheibenzellen getrennt werden und vermehrt und angereichert werden.

Die erfindungsgemäßen Marker können daher vorteilhaft zur Analyse des Anteils von Annulus Fibrosus oder Nucleus Pulposus Zellen in einem Zellgemisch mittels fluoreszenzaktivierter Zellanalyse (fluorescence-activated cell-sorting, FACS, z.B. Becton Dickinson) verwendet werden (Ibrahim SF, van den Engh G. Flow cytometry and cell sorting. Adv Biochem Eng Biotechnol. 2007: 106:19-39.).
Diese etablierte Methode dient zum quantitativen Nachweis der Zielzelle in einem Zellgemisch anhand eines ausgewählten Markes auf Proteinebene (Antigen) mit einem Fluoreszenzmarkierten Antikörper (Antigen-Antikörper-Reaktion). Die gefärbte Zellsuspension wird hydrodynamisch fokussiert und die Zellen werden vereinzelt an einer Detektionseinheit (Laser) vorbeigeführt. Bei positiver Antikörper-Markierung wird ein Fluoreszenzsignal emittiert und somit die Zelle als Zielzelle erkannt. Dadurch errechnet sich der prozentuale Anteil der Zielzelle in einem Zellgemisch und es kann beispielsweise die Reinheit eines Transplantats oder Implantats für die Bandscheibe bestimmt werden.

Als Erweiterung der analytischen Durchflusszytometrie kann die Zellsortierung (z.B. FACS) erfindungsgemäß genutzt werden um Zellen aus einem Zellgemisch zu isolieren. Hierbei werden die Zielzellen nach der Detektionseinheit durch eine bautechnische Erweiterung des FACS-Geräts selektiv in ein Sammelgefäß geleitet. Alternativ, kann die magnetisch-aktivierte Zellsortierung (magnetic-activated-cell-sorting, MACS) angewendet werden bei der Marker-spezifischen Antikörper, die an magnetische Beads gekoppelt sind, zur Erkennung der Zielzellen genutzt werden (z.B. Miltenyi S et al. High gradient magnetic cell separation with MACS. Cytomettry. 1990; 11(2):231-8). Durch Zellsortierung mittels der erfindungsgemäßen Marker können Annulus Fibrosus Zellen und/ oder Nucleus Pulposus Zellen selektioniert und für die Kultivierung angereichert werden.

Dies erlaubt die Isolation von AF-Zellen oder NP-Zellen und Herstellung von Fraktionen oder Implantaten oder Transplantaten bestehend aus AF-Zellen oder NP-Zellen. Die Fraktionen, Implantate oder Transplantate weisen zumindest jeweils mehr als 70 % vorzugsweise mehr als 80 %, besonders bevorzugt mehr als 90 % an AF-Zellen oder NP-Zellen auf.

Weiterhin betrifft die Erfindung ein Mittel, insbesondere ein Arzneimittel, welches die erfindungsgemäßen selektionierten und angereicherten Bandscheibenzellen enthält als auch ein Transplantat oder ein Implantat zur Behandlung und Prophylaxe von Bandscheibenerkrankungen, insbesondere Bandscheibenvorfall oder zur Regeneration der Bandscheibe.

Weiterhin kann das Mittel ausschließlich oder im Wesentlichen aus selektionierten NP-Bandscheibenzellen oder AF-Bandscheibenzellen bestehen. Dies erlaubt vorteilhaft die Transplantation oder Re-Implantation von NP-Bandscheibenzellen oder AF- Bandscheibenzellen in die defekte Bandscheibe, wobei diese in das jeweilige NP- oder AF-Gewebe appliziert werden.

Unter dem Begriff "defekte Bandscheibe" ist eine solche zu verstehen, die eine Bandscheibenerkrankung aufweist, solche degenerativen Erkrankungen, wie Bandscheibenvorfall, Bandscheibenvorwölbung, Bandscheibenprotrusion.

Nachfolgende Beispiele und Figuren dienen zur näheren Erläuterung der Erfindung, jedoch ohne die Erfindung auf diese Beispiele und Figuren zu beschränken.

### Beispiele und Figuren:

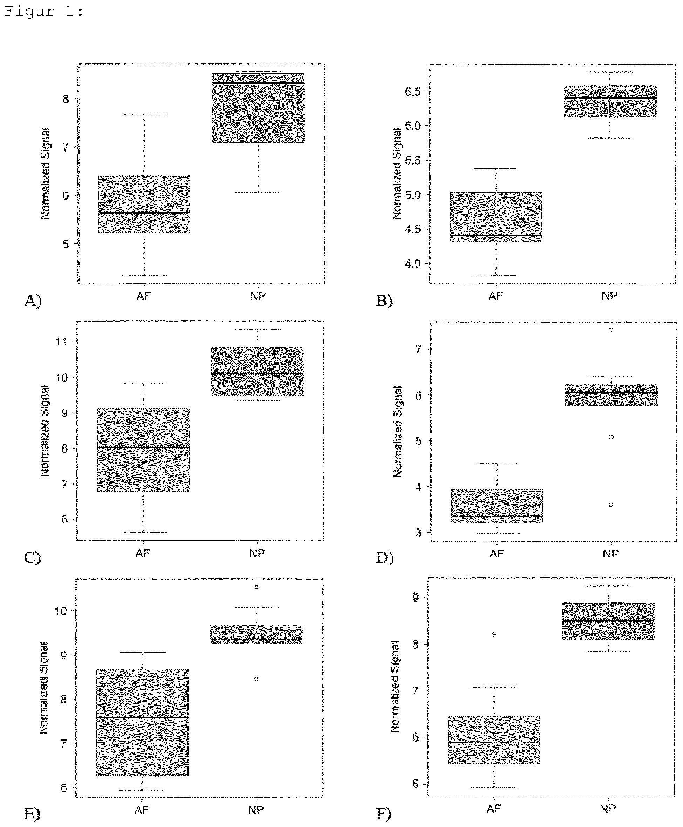

Figur 1 zeigt die differentielle Genexpression des NP-Markerpanels CDKN2B, DEFB1, DSC3, ERFE, SPTLC3 in Bandscheibengewebe. A) ARAP2, B) CDKN2B, C) DEFB1, D) DSC3, E) ERFE, F) SPTLC3 für Annulus Fibrosus (AF) und Nucleus Pulposus (NP). Datengrundlage sind die Genearray-Analysen von AF- und NP-Gewebe (je n=10) mittels der Affymetrix-Technolgie. Die aufgetragenen Werte beschreiben das Signal für das entsprechende Gen relativiert zu allen Genen, deren Expression mit Hilfe von Affymetrix-Chip HGU133P bestimmt wurde (Array-interne rma-Normalisierung mit Affymetrix-Paket in R).

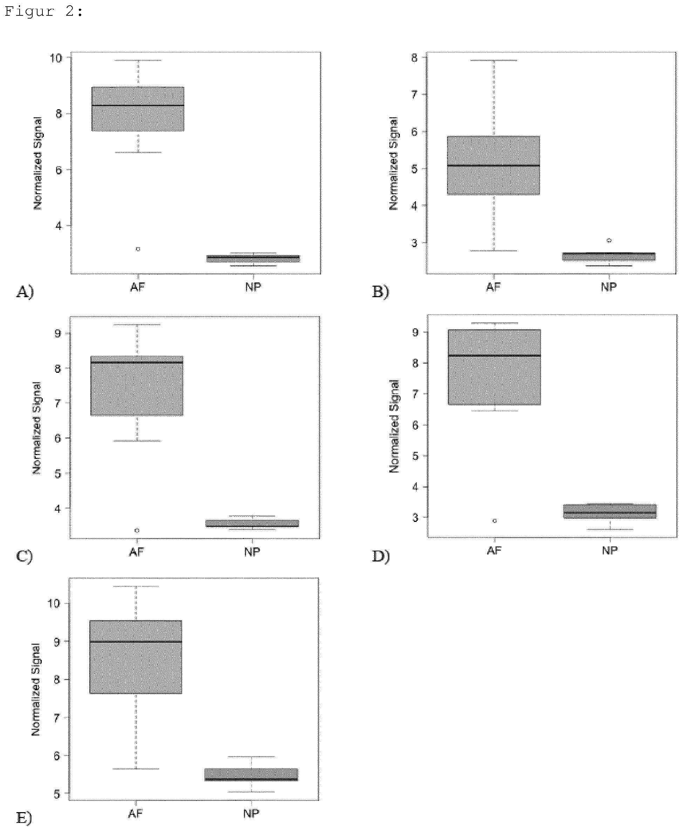

Figur 2 zeigt die differentielle Genexpression des AF-Markerpanels ADGRL4, ANKRD29, EMCN, LDB2, OLFML2A in Bandscheibengewebe. Dargestellt sind die NP-negativen Marker bzw. AF-positiven Marker: A) ADGRL4 (früher ELTD1), B) ANKRD29, C) EMCN, D) LDB2, E) OLFML2A für Annulus Fibrosus (AF) und Nucleus Pulposus (NP). Datengrundlage sind die Genearray-Analysen von AF- und NP-Gewebe (je ne=10) mittels der Affymetrix-Technolgie. Die aufgetragenen Werte beschreiben das Signal für das entsprechende Gen relativiert zu allen Genen, deren Expression mit Hilfe von Affymetrix-Chip HGU133P bestimmt wurde (Array-interne rma-Normalisierung mit Affymetrix-Paket in R).

### Beispiel 1:

Die Bandscheibenzellen werden durch enzymatischen Verdau aus dem Bandscheibengewebe isoliert und anschließend im Monolayer auf adhesiven Zellkulturflaschen in Zellkulturmedium mit humanem Serum bei 37°C, 5% CO2 und angefeuchteter Luft kultiviert. Geeignete Protokolle zur Kultivierung sind beschrieben in:

Ganey T et al, Spine (Phila Pa 1976); 2003 Dec 1;28(23): 2609-20 (co.don AG), Scholz B et al, Euur Cell Mater. 2010 Jul 13; 20:24-36; discussion 36-7 (TETEC AG), Hegewald AA et al, Neurosurg Spine 2011 Feb; 14(2): 273-80 (Transtissue Technologies).

### Beispiel 2:

Zur Analyse der Marker kann die quantitative Real-Time-PCR (qPCR) verwendet werden (Bustin, Absolute quantification of mRNA using real-time reverse transcritption polymerase chain reaction assay, Journal of Molecular Endocrinology (2000) 25, 169-193), nach technischer Umwandlung der mRNA in cDNA (cDNA-Synthese, z.B. Transcriptor First Strand Synthesis Kit von Roche, Mannheim, Deutschland).

Diese etablierte Methode dient dem quantitativen Nachweis von spezifischen Nukleinsäuresequenzen des ausgewählten Zielgens. Bei der qPCR wird die PCR-Amplifikation der cDNA (Template) in Echtzeit verfolgt, d.h. die Akkumulation der amplifizierten Zielsequenz (Amplikon) wird in den einzelnen PCR-Zyklen gemessen. Als Detektionssystem dient beispielsweise das UPL (Universal ProbeLibary) Sondensystem der Firma Roche, Mannheim, Deutschland. Die fluoreszenzmarkierte Sonde wird mit Hilfe des UPL Assay Design Center von Roche ausgewählt. Dies hybridisiert während der PCR-Reaktion sequenzspezifisch zwischen den Primern mit dem Amplikon, wodurch ein Fluoreszenzsignal emittiert wird. Die Signaldetektion wird jeweils am Ende eines jeden PCR-Zyklus durchgeführt. Der Assay setzt sich somit aus einer sequenzspezifischen Sonde (0,1 µM) und den sequenzspezifischen Primern (0,4 µM) zusammen (Tabelle 1). Als Reaktionsmedium wird der LightCyler® 480 Probes Master (Roche) verwendet. Die PCR-Reaktion wird mit einer Template-Konzentration von 0,5ng/µL cDNA unter Verwendung des Geräts LightCycler® 480-II (Roche) durchgeführt.

**Tabelle 1: Beispiel zum Nachweis der Marker auf mRNA-Level. Aufgeführt sind die sequenzspezifischen Sonden und Primerpaare für die einzelnen Zielgene für die qPCR mit dem UPL-Sondensystem von Roche.**

| **Zielgen** | **Sonde** | **Primer 5'** | **Primer 3'** |
|---|---|---|---|
| ADGRL4 | 58 | tccaaaagaccacagagtttga | atgcagcttttctctttggaa |
| ANKRD29 | 44 | gagcaaacatccatgaccaa | ccagcagtaatcgaataacatcc |
| ARAP2 | 61 | agacggagtggatgaccagt | cagctggtggccatatatca |
| CDKN2B | 17 | 9c9999actagtg agaag | ctgcccatcatcatgacct |
| DEFB1 | 86 | tgtctgagatggcctcaggt | gggcaggcagaatagagaca |
| DSC3 | 53 | cagaagcacctggagacgat | gagttgttggtagtttgggtca |
| EMCN | 29 | gaactgcttcaagtgaccattct | aacaagtgaattattagctgcctct |
| ERFE | 80 | ctgctcatctgcatccagtc | ggatggtgaagagctcactgct |
| LDB2 | 53 | gcctgaagacctgcttgttt | gttgttggttgccttgtgg |
| OLFML2A | 19 | acccccaccaccagtctc | ctcacagctcgcctctctg |
| SPTLC3 | 22 | gcagagcttggaaaaagattctc | cagatgcacgatggaacct |

## Patentansprüche

1. Verfahren zur Selektion von humanen Bandscheibenzellen, **dadurch gekennzeichnet, dass** mindestens ein Marker ausgewählt aus der Gruppe
| | |
|---|---|
| ARAP2 | ArfGAP with RhoGAP domain, ankyrin repeat and PH domain 2 |
| CDKN2B | cyclin-dependent kinase inhibitor 2B (p15, inhibits CDK4) |
| DEFB1 | defensin, beta 1 |
| DSC3 | desmocollin 3 |
| ERFE | erythroferrone, frühere Bezeichnung FAM132B |
| SPTLC3 | serine palmitoyltransferase, long chain base subunit 3 |
| ADGRL4 | adhesion G protein-coupled receptor L4 (EGF, latrophilin and seven transmembrane domain containing 1) |
| ANKRD29 | ankyrin repeat domain 29 |
| EMCN | endomucin |
| LDB2 | LIM domain binding 2 |
| OLFML2A | olfactomedin-like 2A |
bestimmt wird.

2. Verfahren zur Selektion von Bandscheibenzellen nach Anspruch 1, wobei die Bandscheibenzellen aus dem Gewebe einer defekten Bandscheibe entnommen werden.

3. Verfahren zum Anreichern der selektionierten Zellen aus Anspruch 1, **dadurch gekennzeichnet, dass** die selektionierten Zellen ex-vivo in einem Kulturmedium kultiviert werden.

4. Verfahren zur Selektion von Bandscheibenzellen nach einem der vorhergehenden Ansprüche, wobei die Bestimmung der Marker die Bestimmung der Zellspezifität oder Zellidentität erlaubt.

5. Verfahren zur Differenzierung zwischen Nucleus Pulposus - Bandscheibenzellen und Annulus Fibrosus - Bandscheibenzellen, wobei eine Bestimmung mindestens eines Markers ausgewählt aus der Gruppe
| | |
|---|---|
| CDKN2B | cyclin-dependent kinase inhibitor 2B (p15, inhibits CDK4) |
| DEFB1 | defensin, beta 1 |
| DSC3 | desmocollin 3 |
| ERFE | erythroferrone, frühere Bezeichnung FAM132B |
| SPTLC3 | serine palmitoyltransferase, long chain base subunit 3 |
und / oder
| | |
|---|---|
| ADGRL4 | adhesion G protein-coupled receptor L4 (EGF, latrophilin and seven transmembrane domain containing 1) |
| ANKRD29 | ankyrin repeat domain 29 |
| EMCN | endomucin |
| LDB2 | LIM domain binding 2 |
| OLFML2A | olfactomedin-like 2A |
erfolgt.

6. Verfahren zum Anreichern der selektionierten Zellen aus Anspruch 5, **dadurch gekennzeichnet, dass** die selektionierten Nucleus Pulposus -Zellen oder Annulus Fibrosus -Zellen ex-vivo in einem Kulturmedium kultiviert werden.

7. Bandscheibenzellen erhältlich nach einem Verfahren gemäß den Patentansprüchen 1 bis 4.

8. Nucleus Pulposus - Bandscheibenzellen oder Annulus Fibrosus -Bandscheibenzellen erhältlich nach einem Verfahren gemäß den Patentansprüchen 5 bis 6.

9. Transplantat oder Implantat enthaltend Zellen nach Anspruch 7 oder Anspruch 8.

10. Mittel, insbesondere Arzneimittel, enthaltend Bandscheibenzellen nach Anspruch 7 oder Anspruch 8 oder ein Transplantat oder ein Implantat nach Anspruch 9 zur Behandlung und Prophylaxe von Bandscheibenerkrankungen, insbesondere Bandscheibenvorfall oder zur Regeneration der Bandscheibe.
